# FASCICULE DE BREVET EUROPEEN

(11) **EP 0 768 865 B1**
(45) Date de publication et mention de la délivrance du brevet: **18.11.1998**
(21) Numéro de dépôt: 95924370.0
(22) Date de dépôt: 27.06.1995
(51) Int. Cl.: A61K 7/07

(54) **SHAMPOOING SEC D'ORIGINE VEGETALE**
TROCKENSHAMPOO PFLANZLICHEN URSPRUNGS
PHYTOGENIC DRY SHAMPOO

(30) Priorité: 28.06.1994 FR 9407942
(43) Date de publication de la demande: 23.04.1997
(73) Titulaire: PIERRE FABRE DERMO-COSMETIQUE, 92100 Boulogne (FR)
(72) Inventeur: JEANJEAN, Michel, F-31320 Castanet-Tolosan (FR); SENEGAS, Nadine, F-31320 Castanet-Tolosan (FR); FABRE, Bernard, F-31450 Belberaud (FR)
(74) Mandataire: Ahner, Francis
(86) Numéro de dépôt international: FR9500853
(87) Numéro de publication internationale: WO9600563

(56) Documents cités:
- EP-A- 0 348 015
- DE-A- 2 806 450
- FR-A- 2 282 257
- FR-A- 2 549 369

## Description

La présente invention a pour objet une nouvelle composition pulvérulente pour shampooing sec.

Les shampooings secs existent depuis de nombreuses années, soit sous forme poudreuse, soit sous forme d'aérosol.

Le shampooing sec permet d'éliminer l'excès de sébum d'une façon rapide, non contraignante et sans mouiller la chevelure. Il agit en captant le sébum par absorption grâce à des poudres choisies pour leurs qualités sébophiles.

Dans l'art antérieur, ces poudres, prises isolément ou associées, pouvaient être d'origine minérale, organique ou synthétique. Cependant, dans la pratique, les shampooings secs proposés ne donnent pas entière satisfaction. En particulier deux inconvénients majeurs sont généralement rencontrés, à savoir un dégraissage insuffisant du cheveu par suite d'une trop faible affinité des poudres vis-à-vis du sébum, et une élimination incomplète des poudres au brossage donnant à la surface de la chevelure un aspect insatisfaisant. En outre, l'interdiction d'utiliser les chlorofluorocarbones dans les aérosols a entraîné des problèmes galéniques complémentaires pour la mise en suspension des poudres et leur distribution en aérosols.

La présente invention a eu pour but de mettre au point une nouvelle composition pour shampooing sec qui offre à la fois une activité nettoyante optimale, une excellente élimination au moment du coiffage et des propriétés physiques qui la rendent parfaitement adaptée à la propulsion par un mélange d'hydrocarbures, dans le cas où l'on désire conditionner le shampooing sous forme aérosol.

En ce qui concerne la propulsion, la nouvelle composition permet notamment d'augmenter significativement le volume du mélange de poudre par rapport au volume du mélange propulseur, de diminuer de manière remarquable les phénomènes de tassement des particules dans la phase liquide, et d'obtenir une homogénéisation facile et efficace des phases solides poudreuses et liquides par simple agitation. Le problème technologique majeur qui se pose quand on passe des chlorofluorocarbones aux hydrocarbures est une baisse substantielle de la densité. Les chlorofluorocarbones présentent en effet une densité supérieure à 1,30 alors que les hydrocarbures présentent une densité voisine de 0,55. Les poudres formulées devront par conséquent présenter des densités inférieures à 0,60. Par ailleurs, les phénomènes d'agglomération entre particules pourront être diminués, voire éliminés, par l'utilisation de mélanges présentant une teneur en humidité la plus basse possible, c'est par exemple le cas des amidons ou des farines.

De plus, une autre caractéristique importante pour le bon fonctionnement de l'aérosol est la finesse particulaire. La poudre sera donc de préférence micronisée pour une parfaite circulation de la poudre à travers tous les orifices du système de diffusion.

Le terme "micronisé" désignera, selon la présente invention, une poudre de granulométrie moyenne calibrée inférieure à environ 100 µm, et de préférence inférieure à environ 50 µm.

En outre, la finesse particulaire, qui est corrélée à la surface spécifique de la poudre, est aussi très importante en ce qui concerne le pouvoir couvrant de la poudre et par conséquent son efficacité en tant qu'agent nettoyant, en particulier à faible dose.

La composition pour shampooing sec selon l'invention qui permet de respecter les critères définis ci-dessus, à savoir le pouvoir dégraissant, la facilité d'élimination par brossage et, dans le cas où elle est utilisée en aérosol, la compatibilité avec la propulsion par un mélange d'hydrocarbures, est la suivante :
- silice sphérique microporeuse,
- β-cyclodextrine,
- amidon modifié, et éventuellement
- une argile,
   dans laquelle la β-cyclodextrine, l'amidon modifié et l'argile seront de préférence micronisés, et l'argile sera de préférence de l'hectorite.

A cette composition on ajoutera avantageusement des matières végétales choisies pour leurs propriétés spécifiques et qui pourront se présenter sous forme :
- de poudres micronisées obtenues par cryobroyage et tamisage, ou bien
- d'extraits adsorbés et vectorisés sur des poudres calibrées, par exemple des amidons.

Sous ces formes les matières végétales seront facilement véhiculables, aussi bien sous la forme de shampooings secs en aérosol, que de poudres, en tenant compte des critères techniques précédemment évoqués, tels que la densité et la finesse particulaire.

La matière végétale sera notamment choisie parmi : l'ortie dioïque, la capucine, l'écorce de quinquina, la farine d'avoine, le bois de panama, le lierre, la camomille, le henné neutre, la saponaire, le noyer, l'hamamélis, ainsi que leurs mélanges.

La composition précédente pourra être utilisée pour un shampooing sec présenté en aérosol. Selon un mode de mise en oeuvre particulier de l'invention, la composition sera la suivante :
- 0,05 à 2 % en poids de silice sphérique microporeuse
- 0,1 à 4 % en poids de β-cyclodextrine
- 1 à 4 % en poids d'amidon modifié, et éventuellement
- 0,1 à 4 % en poids d'une argile.

Le shampooing sec en spray contiendra également au moins un mélange propulseur à base d'hydrocarbures et souvent un polysiloxane lui sera avantageusement ajouté.

La composition précédente pourra également être utilisée pour un shampooing sec en poudre.

Toutes sortes d'adaptations peuvent être effectuées à partir de la composition de base, telles que l'incorporation de principes actifs, parfums, colorants, etc.

La description détaillée faite ci-après montrera l'intérêt particulier de la composition selon l'invention en s'appuyant sur un exemple de préparation de shampooing sec en aérosol, qui n'est en rien limitatif de la présente invention.

### Exemple 1

### Intérêt d'associer la silice sphérique microporeuse aux autres poudres en ce qui concerne la propulsion dans le cas d'un aérosol

La silice sphérique microporeuse donne un mélange plus expansé, d'une tendance au tassement remarquablement diminuée.

En raison de sa finesse extrême (inférieure à 5 µm), la silice sphérique microporeuse influe positivement dans la fluidité et la dispersibilité du mélange final.

Sa forme sphérique parfaite évite les frottements interparticulaires, assurant une meilleure circulation du mélange des poudres à travers la valve et le diffuseur de l'aérosol.

Des tests de vidange effectués sur deux lots de boîtiers aérosols confirment l'intérêt technique d'associer la silice sphérique microporeuse aux autres poudres.

**Tableau 1**

| | Régularité dans la diffusion | Bouchage du système de diffusion |
|---|---|---|
| Shampooing sec renfermant la silice sphérique | Satisfaisante à 100% | Absence de bouchage |
| Shampooing sec sans silice sphérique 30 boîtiers | Satisfaisante à 77% (7 boîtiers défectueux) | 2 bouchages (6,6%) |

### Exemple 2

### Intérêt de la composition en ce qui concerne sa fonction nettoyante et son élimination par brossage

Le pouvoir nettoyant a été évalué de la façon suivante.

Les mèches de cheveux sont d'abord standardisées par lavage puis imprégnées d'un sébum reconstitué.

Le sébum est composé d'un mélange de cholestérol, squalane, acide palmitique, acide oléique, trioléate de glycérol.

La mèche de cheveux est mise en contact de façon homogène avec une quantité de poudre déterminée.

Après élimination par brossage, une extraction par solvant permet de déterminer le sébum résiduel et de quantifier le pouvoir dégraissant de la poudre évaluée.

Cette évaluation quantitative est effectuée par analyse chromatographique en phase gazeuse. Les pourcentages indiqués dans le tableau suivant représentent la quantité de sébum reconstitué qui a été enlevé de son support kératinique.

La facilité d'élimination des poudres a été appréciée de la façon suivante : en "cabine" sur cheveux bruns, l'appréciation tenant compte de l'état du cheveu après brossage.

Les pourcentages indiqués sont issus d'une évaluation approximative sur mèches de cheveux bruns, longueur 15 cm, poids environ 12 g.

**Tableau 2**

| | | % sébum absorbé (1) | % Poudre éliminée (2) |
|---|---|---|---|
| Micro silice sphérique (3) | | 80% | - |
| Amidon modifié | | 45% | - |
| β-cyclodextrines micronisées | | 53% | - |
| Formule shampooing sec avec amidon modifié | 8g | 47% | 83% insuffisant |
| Formule shampooing sec avec amidon modifié | 4g | | |
| microsilice sphérique | 1g | 64% | 94% correct |
| Formule shampooing sec avec amidon modifié | 4g | | |
| microsilice phérique | 1g | | |
| β-cyclodextrines | 0,5g | | |
| micronisées | | 66% | 100%excellent |

L'étude de ce tableau conduit aux observations suivantes :
- La supériorité du pouvoir délipidant (80%) observé sur la silice microporeuse montre combien le rôle joué par la surface spécifique est important. On rappelle que la surface spécifique de la silice microporeuse est de l'ordre de 300 m² au gramme.
- l'association de silice sphérique microporeuse à l'amidon modifié traditionnel augmente de façon surprenante le pouvoir dilipidant de ce dernier. En effet la moyenne pondérée des :
   . pourcentage de sébum éliminé par la silice microporeuse : 80% x 1g de silice
   . pourcentage de sébum éliminé par l'amidon modifié : 47% x 4 g d'amidon conduirait théoriquement à un pourcentage global de sébum éliminé de 54%.
      Les 64% obtenus en réalité constituent donc un résultat particulièrement inattendu de l'invention. Cela a permis de diminuer significativement la quantité totale de poudre à utiliser pour obtenir un nettoyage satisfaisant.
- l'évaluation "cabine" a mis en évidence que la présence de 0,5 à 1 % de β-cyclodextrine dans la formule à base d'amidon induit une parfaite élimination des poudres de la chevelure par simple brossage.

### Exemple 3

### Intérêt de la β-cyclodextrine micronisée par rapport à la β-cyclodextrine standard.

La β-cyclodextrine micronisée calibrée présente un diamètre moyen inférieur à environ 100 microns, et de préférence inférieur à environ 50 microns.

La β-cyclodextrine standard présente un diamètre moyen supérieur à environ 250 microns.

L'évaluation de leur pouvoir dégraissant respectif a été effectuée en suivant la méthode de l'exemple 2, et elle a conduit à l'observation suivante :
β-cyclodextrine micronisée : 53 % de pouvoir délipidant
β-cyclodextrine standard : 41 % de pouvoir délipidant.

Le pouvoir délipidant de la β-cyclodextrine calibrée micronisée peut s'expliquer par un échange entre la phase volatile continue dans sa cavité lipophile et le sébum disposé à la surface du cheveu.

### Exemple 4

### formulation pour un shampooing sec en aérosol

### Phase solide poudreuse

- silice microporeuse sphérique: 0,05 à 2 g
- β-cyclodextrine micronisée: 0,1 à 3 g
- amidon de maïs modifié: 1 à 10 g
- hectorite: 0,1 à 3 g

### Phase liquide

- extrait végétal: QS
- éthanol: QS
- parfum: QS
- décaméthylcyclopentasiloxane: QS
- mélange propulseur à base d'hydrocarbures: QSP 150 ml

La phase solide poudreuse selon cet exemple présente une densité d'environ 0,34 pour la poudre tassée.

## Revendications

1. Composition pulvérulente pour shampooing sec caractérisée en ce qu'elle comprend :
- de la silice microporeuse,
- de la β-cyclodextrine,
- de l'amidon modifié et éventuellement,
- une argile.

2. Composition pulvérulente pour shampooing sec selon la revendication 1, caractérisée en ce que la β-cyclodextrine, l'amidon modifié et l'argile sont micronisés.

3. Composition pulvérulente pour shampooing sec selon l'une des revendications 1 ou 2, caractérisée en ce que l'argile est de l'hectorite.

4. Shampooing sec caractérisé en ce qu'il se présente sous forme d'un aérosol et en ce qu'il contient au moins un mélange propulseur à base d'hydrocarbures et la composition pour shampooing sec selon les revendications 1 à 3 dans les proportions suivantes :
- 0,05 à 2 % en poids de silice sphérique microporeuse,
- 0,1 à 4 % en poids de β-cyclodextrine,
- 1 à 4 % en poids d'amidon modifié, et éventuellement
- 0,1 à 4 % en poids d'une argile.

5. Shampooing sec selon la revendication 4, caractérisé en ce qu'il contient également un polysiloxane.

6. Shampooing sec caractérisé en ce qu'il se présente sous forme de poudre et en ce qu'il contient au moins la composition pour shampooing sec selon l'une quelconque des revendications 1 à 3.

7. Shampooing sec selon l'une quelconque des revendications 4 à 6, caractérisé en ce qu'il comprend en outre de la matière végétale.

8. Shampooing sec selon la revendication 7, caractérisé en ce que la matière végétale est choisie parmi l'ortie dioïque, la capucine, l'écorce de quinquina, la farine d'avoine, le bois de panama, le lierre, la camomille, le henné neutre, la saponaire, le noyer, l'hamamélis, ainsi que leurs mélanges.

9. Shampooing sec selon les revendications 7 ou 8, caractérisé en ce que la matière végétale se présente sous forme de poudre micronisée.

10. Shampooing sec selon l'une quelconque des revendications 7 à 9, caractérisé en ce que la matière végétale se présente sous forme d'extraits adsorbés et vectorisés sur une poudre calibrée, notamment de l'amidon.

11. Shampooing sec en aérosol selon les revendications 4 ou 5, caractérisé en ce qu'il est composé
d'une phase solide poudreuse comprenant :
sllice microporeuse sphérique 0,05 à 2 g
β-cyclodextrine micronisée 0,1 à 3 g
amidon de maïs modifié 1 à 10g
hectorite 0,1 à 3g
et d'une phase liquide comprenant :
extrait végétal QS
éthanol QS
parfum QS
décaméthylcyclopentasiloxane QS
mélange propulseur à base d'hydrocarbures QSP 150 ml.

## Claims

1. Pulverulent dry-shampoo composition, characterized in that it comprises:
- microporous silica,
- β-cyclodextrin,
- modified starch, and optionally
- a clay.

2. Pulverulent dry-shampoo composition according to Claim 1, characterized in that the β-cyclodextrin, the modified starch and the clay are micronized.

3. Pulverulent dry-shampoo composition according to either of Claims 1 and 2, characterized in that the clay is hectorite.

4. Dry shampoo characterized in that it is in the form of an aerosol and in that it contains at least one hydrocarbon-based propellent mixture and the dry-shampoo composition according to Claims 1 to 3 in the following proportions:
- 0.05 to 2% by weight of microporous spherical silica,
- 0.1 to 4% by weight of β-cyclodextrin,
- 1 to 4% by weight of modified starch, and optionally
- 0.1 to 4% by weight of a clay.

5. Dry shampoo according to Claim 4, characterized in that it also contains a polysiloxane.

6. Dry shampoo, characterized in that it is in powder form and in that it contains at least the dry-shampoo composition according to any one of Claims 1 to 3.

7. Dry shampoo according to any one of Claims 4 to 6, characterized in that it also comprises plant material.

8. Dry shampoo according to Claim 7, characterized in that the plant material is chosen from dioic nettle, nasturtium, cinchona bark, oatmeal, panama wood, ivy, camomile, neutral henna, saponin, walnut and hamamelis, as well as mixtures thereof.

9. Dry shampoo according to Claim 7 or 8, characterized in that the plant material is in the form of micronized powder.

10. Dry shampoo according to any one of Claims 7 to 9, characterized in that the plant material is in the form of extracts adsorbed and vectorized onto a calibrated powder, in particular starch.

11. Dry shampoo in aerosol form, according to Claim 4 or 5, characterized in that it is composed of a powdery solid phase comprising:
spherical microporous silica 0.05 to 2 g
micronized β-cyclodextrin 0.1 to 3 g
modified corn starch 1 to 10 g
hectorite 0.1 to 3 g
and of a liquid phase comprising:
plant extract qs
ethanol qs
fragrance qs
decamethylcyclopentasiloxane qs
hydrocarbon-based propellent mixture qs for 150 ml.

## Patentansprüche

1. Pulverförmige Zusammensetzung für Trockenshampoo, dadurch charakterisiert, daß sie umfaßt:
- mikroporöse Kieselerde
- β-Cyclodextrin
- modifizierte Stärke und gegebenenfalls
- einen Ton.

2. Pulverförmige Zusammensetzung für Trockenshampoo nach Anspruch 1, dadurch charakterisiert, daß das β-Cyclodextrin, die modifizierte Stärke und der Ton mikronisiert sind.

3. Pulverförmige Zusammensetzung für Trockenshampoo nach einem der Ansprüche 1 und 2, dadurch charakterisiert, daß der Ton Hektorit ist.

4. Trockenshampoo, dadurch charakterisiert, daß es in Form eines Aerosols vorliegt und mindestens eine Treibmittelmischung auf Kohlenwasserstoffbasis und die Zusammensetzung als Trockenshampoo nach den Ansprüchen 1 bis 3 in den folgenden Anteilen enthält:
- 0,05 bis 2 Gew.-% sphärische mikroporöse Kieselerde
- 0,1 bis 4 Gew.-% β-Cyclodextrin
- 1 bis 4 Gew.-% modifizierte Stärke, und gegebenenfalls
- 0,1 bis 4 Gew.-% eines Tons.

5. Trockenshampoo nach Anspruch 4, dadurch charakterisiert, daß es auch ein Polysiloxan enthält.

6. Trockenshampoo, dadurch charakterisiert, daß es in Pulverform vorliegt un daß es mindestens die Zusammensetzung für Trockenshampoo nach einem der Ansprüche 1 bis 3 enthält.

7. Trockenshampoo nach einem der Ansprüche 4 bis 6, dadurch charakterisiert, daß es zusätzlich pflanzliches Material enthält.

8. Trockenshampoo nach Anspruch 7, dadurch charakterisiert, daß das pflanzliche Material ausgewählt wird aus diözischer Brennessel, Kapuzinerkresse, Chinarinde, Hafermehl, Panamaholz, Efeu, Kamille, neutralem Henna, Seifenkraut, Walnußbaum, Hamamelis und ihren Gemischen.

9. Trockenshampoo nach den Ansprüchen 7 oder 8, dadurch charakterisiert, daß das pflanzliche Material als mikronisiertes Pulver vorliegt.

10. Trockenshampoo nach einem der Ansprüche 7 bis 9, dadurch charakterisiert, daß das pflanzliche Material in Form von Extrakten vorliegt, die auf kalibriertem Pulver adsorbiert und vektorisiert sind, insbesondere auf Stärke.

11. Aerosol-Trockenshampoo nach den Ansprüchen 4 oder 5, dadurch charakterisiert, daß es zusammengesetzt ist aus
einer festen pulverigen Phase, umfassend:
sphärische mikroporöse Kieselerde 0,05 bis 2 g
mikronisiertes β-Cyclodextrin 0,1 bis 3 g
modifizierte Maisstärke 1 bis 10 g
Hektorit 0,1 bis 3 g
und einer flüssigen Phase, umfassend:
Pflanzenextrakt QS
Ethanol QS
Duftstoff QS
Decamethylcyclopentasiloxan QS
Treibmittelmischung auf Kohlenwasserstoffbasis QSP 150 ml.
